(19) Europäisches Patentamt
European Patent Office
Office européen des brevets

(11) Numéro de publication: **0 038 251**
**A1**

# (12) DEMANDE DE BREVET EUROPEEN

(21) Numéro de dépôt: **81400547.6**

(22) Date de dépôt: **03.04.81**

(51) Int. Cl.³: **C 07 H 3/06**, C 07 H 3/08, C 07 H 15/18, G 01 N 33/80

(30) Priorité: **03.04.80 FR 8007614**

(43) Date de publication de la demande: **21.10.81**
**Bulletin 81/42**

(84) Etats contractants désignés: **BE CH DE GB IT LI NL SE**

(71) Demandeur: **ANVAR Agence Nationale de Valorisation de la Recherche, 43, rue Caumartin, F-75436 Paris Cedex 09 (FR)**

(72) Inventeur: **Maman, Michel, 2, rue Cavès, F-92100 Montrouge (FR)**
Inventeur: **Ropars, Claude, 17 bis, rue Ste Geneviève, F-94400 Vitry (FR)**
Inventeur: **Sinay, Pierre, 5, rue Jacques Monod, F-45100 Orleans (FR)**

(74) Mandataire: **Peaucelle, Chantal et al, Cabinet Plasseraud 84, rue d'Amsterdam, F-75009 Paris (FR)**

## (54) Nouveaux dérivés d'osides, leur préparation et leurs applications biologiques.

(57) Dérivés d'osides possédant des propriétés antigéniques, comprenant un motif galactopyranose, dans lequel le groupe hydroxyle en position 1 est substitué par une chaîne comprenant un groupe éther intercalaire et un groupement fonctionnel azoté en bout de chaîne.

Ces osides sont notamment utiles pour préparer des immunoabsorbants.

EP 0 038 251 A1

Nouveaux dérivés d'osides, leur préparation et leurs applications biologiques.

L'invention est relative à de nouveaux dérivés d'osides, possédant des propriétés de groupes sanguins, en particulier à spécificité B, ou constituant des intermédiaires de grand intérêt pour l'obtention de produits possédant de telles propriétés.

Elle concerne également leur préparation et leurs applications biologiques, plus spécialement en immunohématologie.

L'invention concerne plus particulièrement des dérivés de substitution du trisaccharide B.

Ce trisaccharide ou 2-O-(α-L-fucopyranosyl)-3-O-(α-D-galactopyranosyl)-D-galactopyranose de formule :

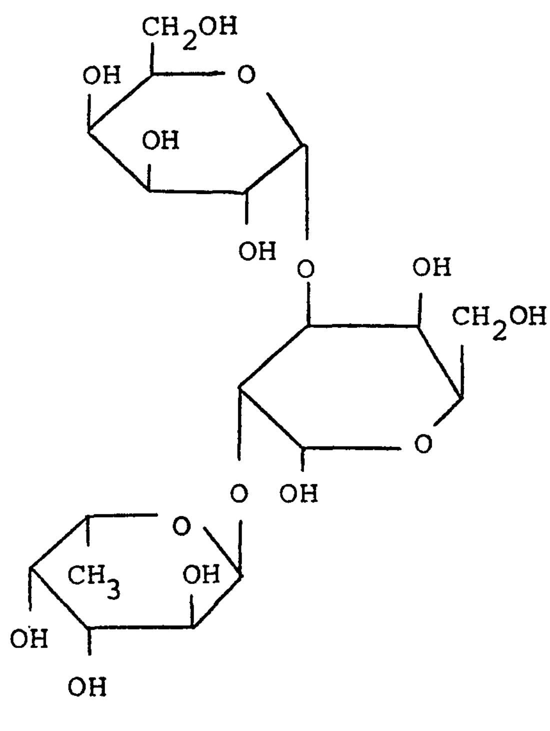

(I)

constitue le déterminant antigénique B de groupe sanguin humain.

En raison de cette spécificité antigénique, le trisaccharide B trouve des applications de grande

importance dans divers secteurs biologiques, en particulier en immunohématologie et plus spécialement dans la détermination des groupes sanguins.

Mais si ce trisaccharide B ou un dérivé d'oside possédant une spécificité antigénique B peut être utilisé tel quel, par exemple en tant que réactif antigénique ou pour préparer un sérum d'essai, il doit contenir une chaîne de substitution, plus particulièrement un bras de couplage pour pouvoir être fixé sur un support, afin de former, plus spécialement, un antigène artificiel ou un immunoabsorbant.

Le problème est donc de disposer d'un dérivé osidique à partir duquel les diverses applications souhaitées pourront être aisément mises en oeuvre.

Mettant à profit les avantages du procédé pour la préparation de dérivés d'osides décrit dans la demande de brevet FR N° 78 10558 au nom de la demanderesse, les inventeurs ont étudié de manière approfondie diverses chaînes de substitution.

Les travaux effectués ont permis de constater qu'il était possible d'obtenir des chaînes de substitution spécifiques qui peuvent être aisément modifiées, et en conséquence particulièrement utiles pour les applications biologiques désirées. Plus spécialement, les inventeurs ont constaté qu'il était possible d'obtenir des osides substitués qui permettent d'accéder à des supports solides insolubles de grande valeur, particulièrement utiles pour la préparation d'immunoabsorbants.

L'invention a donc pour but de fournir de nouveaux dérivés d'osides, en particulier des dérivés de substitution du trisaccharide B, pouvant être utilisés tels quels dans les applications biologiques désirées ou pouvant être modifiés aisément selon l'application envisagée.

Elle vise également à fournir des réactifs antigéniques préparés à l'aide de ces osides ainsi que des immunoabsorbants obtenus par couplage de ces osides sur des supports particuliers.

L'invention se rapporte plus particulièrement à des osides comprenant une structure osidique à spécificité de groupe sanguin avec un motif galactopyranose dont la position 1 est substituée par un groupe -OA, dans lequel A comprend au moins un radical éther et possède un groupe fonctionnel terminal azoté.

Les dérivés d'oside de l'invention sont plus particulièrement caractérisés par le fait que dans ledit motif galactopyranose, A représente une chaîne comprenant un radical alcoxy-alcoylamine, alcoxy-alcoylamide ou alcoxy-alcoylazide, dans lequel les groupes alcoxy et alcoyle renferment 3 à 15 atomes de carbone, de préférence de 4 à 10, avantageusement 6.

Les osides de ce type avec une chaîne terminale amino et possédant des groupes alcoxy et alcoyle renfermant chacun 3 atomes de carbone sont particulièrement préférés.

Les motifs galactopyranose définis ci-dessus font avantageusement partie d'un oside comprenant une chaîne trisaccharidique linéaire ou branchée comportant un motif fucopyranose et un autre motif galactopyranose, avec une liaison 1,2-cis, en particulier possédant la structure du trisaccharide B.

Dans ces chaînes trisaccharidiques, les groupes hydroxyle des différents motifs ose sont libres ou protégés par des groupes de blocage choisis parmi les groupes stables qui ne sont pas réactifs dans les conditions usuelles de synthèse des osides et qui peuvent être facilement éliminés dans des conditions ménagées compatibles avec le maintien de la structure osidique.

Les trisaccharides correspondant aux caractéristiques définies ci-dessus, et plus spécialement les

trisaccharides branchés constitués par un motif galactopyranose contenant un substituant A tel que défini ci-dessus, et qui est simultanément substitué par un autre motif galactopyranose et par un motif fucopyranose sont préférés.

En particulier, l'invention concerne des trisaccharides branchés de formule II :

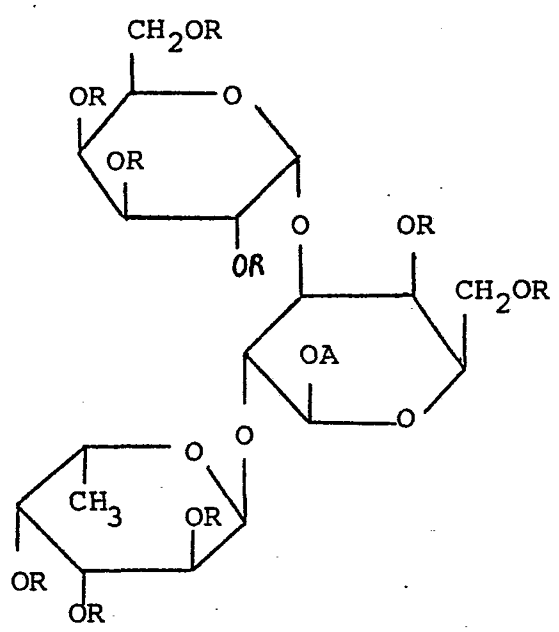

(II)

dans laquelle les substituants R, identiques ou différents les uns des autres, représentent des groupements de protection de radicaux hydroxyle, le cas échéant avec un substituant voisin, et sont choisis parmi des groupes stables, non réactifs dans les conditions habituelles de synthèse osidique et facilement éliminables dans des conditions douces compatibles avec le maintien de la structure osidique, en particulier par des groupes constituant avec l'atome d'oxygène de l'hydroxyle des éthers benzyliques ou des acétals benzylidéniques, ou représentent un atome d'hydrogène,

et A représente un radical alcoxy-alcoylamine, alcoxy-alcoylamide ou alcoxy-alcoylazide, dans lequel les

groupes alcoxy et alcoyle comprennent 3 à 15 atomes de carbone, de préférence 4 à 10, avantageusement 6.

De préférence, les groupes alcoxy et alcoyle renferment chacun 3 atomes de carbone.

D'autres produits préférés comprennent un groupe alcoxy avec 3 atomes de carbone, et un groupe alcoyle avec au moins 5 atomes de carbone.

On notera qu'en raison de sa nature hydrophile, la fonction éther intercalaire dans A est particulièrement précieuse pour les diverses applications biologiques des osides de l'invention. Ce groupement fonctionnel éther peut cependant être modifié si on le souhaite et transformé en un autre radical.

Compte tenu de leurs possibilités d'application pour la constitution d'immunoabsorbants, les osides dans lesquels A est constitué par une chaîne de substitution à terminaison amino, spécialement par un groupe amino primaire, sont particulièrement préférés. Comme il sera indiqué ci-dessous, de telles chaînes permettent avantageusement l'utilisation de supports solides insolubles permettant de disposer d'immunoabsorbants à pouvoir d'absorption spécifique élevé et de grande stabilité.

Plus spécialement, ces chaînes permettent la fixation de dérivés d'osides à spécificité de groupes sanguins B sur des supports solides conduisant à des immunoabsorbants comportant des sites de fixation -OH, -COOH ou $-CONH_2$ , dépourvus ou pratiquement dépourvus d'absorption non spécifique.

Une autre famille d'osides préférés comprend un radical azido en bout de chaîne. De telles azides possèdent un double avantage. D'une part, il s'agit de composés stables. D'autre part, ils permettent l'obtention aisée de chaînes de substitution avec un groupe terminal amino particulièrement précieux pour la constitution d'immunoabsorbants de l'invention.

Selon un autre mode de réalisation, A comprend en outre un ou plusieurs groupes insaturés, en particulier des groupes éthyléniquement insaturés.

Les osides correspondants sont avantageusement utilisables tels quels en tant que réactifs biologiques. En outre, par un jeu de substitution de l'insaturation éthylénique, ils permettent avantageusement l'introduction de groupes fonctionnels ou l'élongation de la chaîne comme souhaité.

Dans un autre groupe d'osides de l'invention, A comprend en outre un ou plusieurs groupes -OH, ces groupes -OH étant ou non protégés.

Dans encore un autre groupe, A comprend en plus d'au moins l'un des dix groupes éther ou à la place d'un tel groupe, au moins un groupe azoté intercalaire, en particulier un groupe amino.

Les osides de l'invention comprenant des motifs galactopyranose possédant une chaîne de substitution telle que définie ci-dessus sont avantageusement synthétisés à partir d'un allyl-2-O-galactopyranose.

Dans cette série particulière d'osides possédant une stéréospécificité donnée, il s'est avéré que la réactivité du groupe allyle présente une grande souplesse et offre de grandes possibilités pour l'introduction de groupes fonctionnels précieux vis-à-vis des applications envisagées.

Les osides comprenant ladite structure saccharidique, en particulier les trisaccharides eux-mêmes de formule II ci-dessus, sont alors avantageusement préparés à partir des allyl-trisaccharides décrits dans la demande de brevet FR N° 78 10558, qui répondent à la formule :

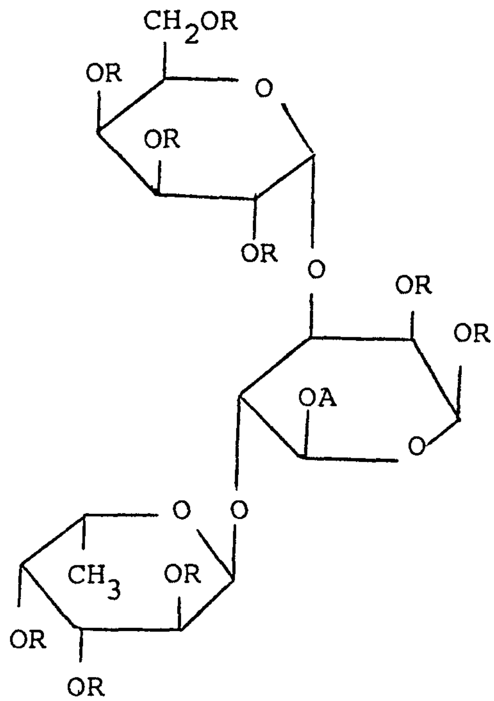

dans laquelle R présente les significations indiquées ci-dessus, et A représente un groupe allyle.

La mise en oeuvre du dérivé allyle, en tant que matériau de départ, présente de nombreux avantages.

Tout d'abord, ce composé peut être facilement obtenu en mettant en oeuvre le procédé décrit dans ladite demande de brevet FR, et ce avec des rendements élevés et un degré de pureté élevé.

En outre, il s'est avéré que la réactivité du groupe allyle, qui peut donc être mise à profit même dans cette série particulière d'osides, permet l'articulation d'un grand nombre de réactions et par là l'obtention de chaînes de longueur et de nature modulables, à caractère hydrophile précieux pour les applications évoquées ci-dessus.

Pour l'allongement de la chaîne, on a recours aux réactions classiques de la synthèse organique, qui seront aisément mises en oeuvre par l'homme de l'art en fonction de la chaîne souhaitée.

Il est notamment avantageux, pour allonger la

chaîne allylique, de soumettre le dérivé allyle à une réaction d'hydroboration, en utilisant un réactif tel que le 9-borabicyclo[3.3.1]nonane, puis à l'action d'une base.

On obtient alors une chaîne correspondante terminée par un groupement fonctionnel alcool. On notera que ce groupe peut être aisément éthérifié et permet l'introduction d'un groupe éther intercalaire dans la chaîne de substitution A.

En vue des applications où il apparaît souhaitable de disposer d'une chaîne servant de bras de couplage pour la fixation sur un support, on traire la chaîne à groupement alcool précédente par un halogénure d'allyle pour introduire un nouveau groupement allyle qui sera à son tour avantageusement soumis à une réaction d'hydroboration.

Ce jeu de réactions permet d'augmenter, comme souhaité, la longueur de la chaîne de substitution et de disposer en bout de chaîne de groupements fonctionnels permettant par leur réactivité une meilleure mise à profit des propriétés des osides de l'invention.

Parmi les diverses possibilités de traitement offertes, il est avantageux de recourir à une tosylation puis de faire réagir le dérivé tosylé avec un azide.

Comme déjà indiqué, cette famille d'osides possède plus spécialement des propriétés antigéniques B et sont avantageusement utilisables dans diverses réactions immunologiques en remplacement du déterminant antigénique B de groupe sanguin.

L'invention fournit ainsi de précieux réactifs biologiques utilisables sous forme immobilisée, mais également tels quels. L'étude de ces dérivés sous leur forme libre a montré notamment leur capacité élevée de neutralisation des anticorps anti-B notamment des hémolysines anti-B. Ces dérivés sont donc avantageusement utilisables pour la détection et la neutralisation des

hémolysines anti-B en remplacement des substances de groupes sanguins isolées à partir de mucine d'estomac de porc ou de cheval.

Il est également possible de les immobiliser sur des supports solides afin de constituer des immuno-adsorbants.

Dans ce type d'application, les chaînes de substitution ci-dessus s'avèrent particulièrement précieuses.

Elles possèdent en effet un groupe fonctionnel en bout de chaîne qui permet la fixation de l'oside sur un support solide et constitue, à cet égard, un bras écarteur spécialement intéressant. Ce bras, de nature hydrophile, qui possède, avantageusement, une fonction éther intercalaire, c'est-à-dire au sein de la chaîne, se termine, dans un mode de réalisation préféré de l'invention, par un groupe amino. Cette disposition permet le couplage de l'oside sur des supports solides, de grande stabilité, comportant des sites de fixation -OH ou -COOH, ou $-CONH_2$.

Les recherches approfondies effectuées dans ce domaine par les inventeurs ont révélé à cet égard l'intérêt particulier de supports à base de polymères renfermant des polysaccharides.

Il est alors possible d'accéder à des supports permettant l'élaboration d'immunoadsorbants dotés d'une capacité d'absorption d'anticorps anti-B élevée et d'une manière tout spécialement avantageuse ne donnant pas lieu à des absorptions non spécifiques.

L'étude de tels supports a montré qu'ils sont particulièrement avantageux pour le couplage avec les osides de l'invention, par l'intermédiaire des chaînes de substitution définies ci-dessus, et conduisent à des immunoabsorbants possédant les caractéristiques d'absorption recherchées. Parmi ces supports à base de polysaccharides, il est apparu que ceux à base de cellulose sont particulièrement appropriés.

Plus spécialement, on a constaté que des supports renfermant de la cellulose en combinaison avec d'autres dérivés tels que la lignine sont d'un grand intérêt pour les applications recherchées.

Des supports de ce type préférés sont constitués par la rafle de maïs.

D'une manière avantageuse, la mise en oeuvre de supports de ce type, plus spécialement à base de rafle de maïs, pour le couplage avec les osides de l'invention, permet de disposer d'immunoabsorbants ne donnant pas lieu à des absorptions non spécifiques mais dotés de capacités d'absorption spécifique très élevées.

L'invention vise donc également l'utilisation des supports définis ci-dessus, en particulier de la rafle de maïs, pour le couplage avec les dérivés d'osides de l'invention, aux fins d'élaboration d'immunoabsorbants.

D'autres supports comportant les dites de fixation ci-dessus s'avèrent intéressants du fait de leur pouvoir d'absorption élevé.

Ainsi, des polymères renfermant ou constitués par des polysaccharides tels que de l'agarose ou de l'agarose modifié par réticulation de ses chaînes polysaccharidiques, tel que celui commercialisé sous la marque Sepharose par Pharmacia sont avantageusement utilisables pour le couplage avec les osides de l'invention.

D'autres supports appropriés comprennent des polymères mixtes renfermant, outre les polysaccharides ci-dessus, d'autres dérivés polymères tels que des polyacrylamides. Comme support de ce type convenant dans le cadre de l'invention, on peut citer ceux commercialisés sous les marques ULTROGEL ou encore MAGNOGEL par IBF (Industrie Biologique Française).

D'une manière générale, afin d'empêcher toute fixation non spécifique, le produit de couplage est avantageusement mis en suspension dans une solution

permettant de saturer les sites de fixations encore libres.

Cette mise en suspension est par exemple effectuée dans une solution protéique.

Pour le couplage des produits de l'invention sur les supports du type évoqué ci-dessus, on a avantageusement recours aux méthodes générales utilisées en chromatographie d'affinité.

Selon les techniques courantes, le support solide sur lequel on souhaite immobiliser le dérivé d'oside est au préalable activé. Cette activation peut être réalisée selon différents moyens, par exemple à l'aide de bromure de cyanogène, de carbonyldiimidazole, d'hydrazine ou de glutaraldéhyde ou tout autre produit connu à cet effet et équivalent.

Un support tel que la rafle de maïs est avantageusement activé à l'aide de carbonyldiimidazole.

Il apparaît intéressant de réaliser cette réaction d'activation en milieu solvant organique. Un solvant particulièrement approprié est constitué par le dioxanne.

Ce solvant est avantageusement également utilisé pour laver au préalable la rafle.

De préférence, on soumet au préalable la rafle à un prétraitement afin d'éliminer les fines et de disposer d'un produit ne donnant pas lieu à un relargage.

Le couplage du dérivé d'oside sur le support est effectué en mettant avantageusement en oeuvre de 50 à 500 nM de dérivés d'oside par g de support, de préférence environ 200 nM.

On laisse le mélange en contact, avantageusement sous agitation, le temps nécessaire pour obtenir la fixation souhaitée.

Cette durée peut être variable selon le type de support et sera aisément déterminée par des opérations de routine. A titre indicatif, il est apparu avantageux

de laisser les réactifs en contact durant environ 12 à 24 heures à + 4°C, de préférence pendant environ 14 heures.

En opérant à température ambiante, cette durée peut être de l'ordre de 1 à 4 heures, de préférence de 2 heures environ.

Cette phase de couplage est avantageusement réalisée en milieu basique. Avec des supports du type ULTROGEL et MAGNOGEL, on opère avantageusement à un pH de l'ordre de 8 à 9,5. Divers tampons, excepté ceux contenant des groupements amine primaire, sont utilisables à cet effet notamment des tampons de bicarbonate de sodium, contenant éventuellement du chlorure de sodium.

Dans le cas de l'utilisation de rafle de maïs, l'expérience a montré qu'il est avantageux d'effectuer le couplage dans de la soude.

Afin de bloquer les groupements réactifs du support qui n'auraient pas réagi avec le dérivé d'oside, on traite avantageusement le produit de couplage avec une solution d'éthanolamine.

A cet égard, il apparaît avantageux de soumettre le mélange réactionnel à agitation, à pH 8. En opérant à température ambiante, on laisse la réaction s'effectuer durant 1 à 2 heures environ.

A la place de l'éthanolamine, on peut utiliser, notamment, du tampon Tris-HCl pH 8, de la glycine, de la DL lysine, de l'acide glutamique, de la glucosamine à 0,2 M et pH 8 ou tout autre produit équivalent.

L'addition de NaCl, 0,5 M favorise l'élimination des charges.

Pour saturer éventuellement les charges qui auraient pu échapper au traitement précédent, on a avantageusement recours à un traitement complémentaire comprenant la mise en contact du produit de couplage avec une solution tampon contenant de la sérum albumine

bovine ou humaine ou encore du sérum AB, et/ou le lavage du produit de couplage successivement avec plusieurs tampons avantageusement avec un tampon phosphate 0,1 M, de pH avantageusement de l'ordre d'au moins 8, de préférence entre 8 et 8,5, contenant 0,5 M de chlorure de sodium, puis un tampon acétate 0,1 M de pH inférieur à 5, avantageusement de l'ordre de 4, contenant 0,5 M de NaCl.

Pour la conservation des produits de couplage, on ajoute avantageusement du merthiolate ou de l'azide de sodium aux suspensions de ces produits dans un tampon de pH neutre.

L'invention fournit donc des immunoabsorbants de synthèse particulièrement précieux, permettant l'élaboration de sérums-tests à spécificité B ou dépourvus d'anti-corps anti-B qu'on désire éliminer.

Les sérums-tests de départ utilisés dans ces applications sont des réactifs de laboratoire mis en oeuvre pour déterminer les groupes sanguins. Ils peuvent être d'origine sanguine humaine ou animale ou formés de composés de types lectines ou protectines d'origine animale.

On mesurera l'intérêt de tels immunoabsorbants qui permettent d'éviter des absorptions répétées sur des hématies particulières, qui font parfois défaut pour les applications transfusionnelles.

L'absorption des anti-B présents dans les sérums-tests anti-D est à cet égard particulièrement intéressante et permet d'envisager l'économie de grandes quantités d'hématies B rhésus négatif.

Ces immunoabsorbants peuvent être utilisés pour résoudre d'autres problèmes où intervient une fréquence encore plus rare de combinaisons de systèmes de groupe sanguin.

Par exemple, certains sujets appartiennent au groupe exceptionnel négatif pour l'antigène cellano

(système Kell) et sont susceptibles de développer un anti-corps anticellano pouvant être utilisé comme sérum test pour l'antigène cellano. Selon le processus de détermination classique de groupes sanguins, les anticorps naturels anti-B de ces sujets sont absorbés à l'aide de globules rouges d'un sujet de groupe B, cellano négatif. Si l'on considère qu'une telle combinaison ne se trouve que 1,7 fois sur 10.000, on mesure l'intérêt indéniable de l'invention.

L'intérêt des immunoabsorbants de l'invention est d'autant plus grand qu'ils sont régénérables. Ainsi, après élution en milieu acide des anti-B fixés sur les immunoabsorbants et concentration, on élimine les anticorps anti-B et l'on dispose des immunoabsorbants qui peuvent être à nouveau utilisés. Les anti-B sont alors récupérés avec des titres élevés.

L'invention fournit donc des sérums-tests à spécificité élevée de grand intérêt pour la transfusion sanguine.

De plus, l'absence d'absorption non spécifique qui peut être atteinte avec les immunoabsorbants de l'invention constitue un élément fondamental pour l'utilisation dans les procédés d'épuration par circulation extra-corporelle et pour la préparation de produits sanguins à usage thérapeutique du type cryoprécipité ou analogue comme les concentrés de super VIII obtenus par purification de cryoprécipités ou d'une manière générale tout produit sanguin contenant des anticorps anti-B qu'on souhaite éliminer.

D'une manière générale, les osides de l'invention, plus spécialement les trisaccharides définis ci-dessus, sont utilisables in vitro et en procédé d'épuration par circulation extra-corporelle grâce à leur innocuité et à leur non immunogénicité, et ce dans les diverses réactions immunologiques mettant en jeu les propriétés du déterminant antigénique B.

Ils sont également applicables en tant que substances d'immunisation animale et pour certains d'entre eux chez l'homme.

D'autres caractéristiques et avantages de l'invention apparaîtront dans la suite de la description donnée dans les exemples.

Les points de fusion donnés dans ces exemples sont mesurés dans un tube capillaire au moyen d'un appareil Büchi et ne sont pas corrigés. Les pouvoirs rotatoires sont déterminés avec, comme polarimètre, le modèle 141 commercialisé par Perkin-Elmer. Les spectres infrarouges (IR) sont enregistrés à l'aide d'un spectrophotomètre IRA-1 de Jouan-Jasco et les spectres de résonance magnétique nucléaire (RMN) à l'aide d'un spectromètre Perkin-Elmer R-32 (90 MHz). En ce qui concerne les résultats relatifs à la RMN, on indique les déplacements chimiques (δ) par rapport au tétraméthylsilane interne ; les protons de l'unité L fucopyranose sont affectés d'un indice prime, ceux de l'unité D-galactopyranose non réductrice d'un indice seconde. Les chromatographies sur colonne sont effectuées au moyen de gel de silice Merck (granulométrie 0,063-0,200 mm).

EXEMPLE 1.

Préparation du γ[(γ-azido)propyloxy]propyl-2-O-(2,3,4-tri-O-benzyl-α-L-fucopyranosyl)-3-O-(2,3,4,6-tétra-O-benzyl-α-D-galactopyranosyl)-4,6-O-benzylidène-β-D-galactopyranoside, de formule XXV :

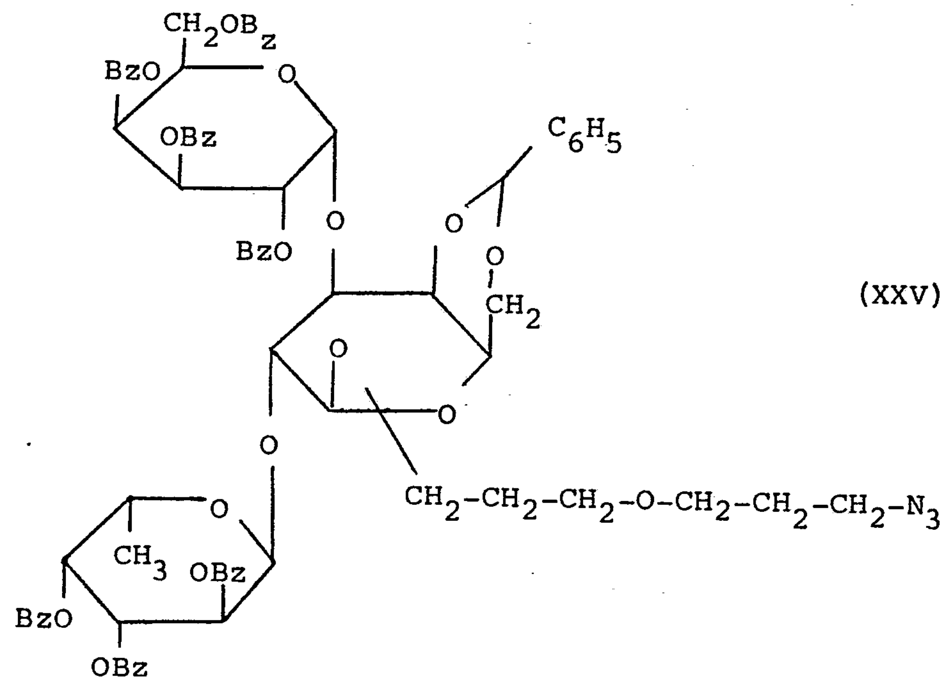

(XXV)

Dans une première étape a), on soumet le trisaccharide XXIII (c'est-à-dire le γ[(γ-hydroxy)propyloxy] propyl-2-O-(2,3,4-tri-O-benzyl-α-L-fucopyranosyl)-3-O-(2,3,4,6-tétra-O-benzyl-α-D-galactopyranosyl)-4,6-O-benzylidène-β-D-galactopyranoside) de formule XXIII :

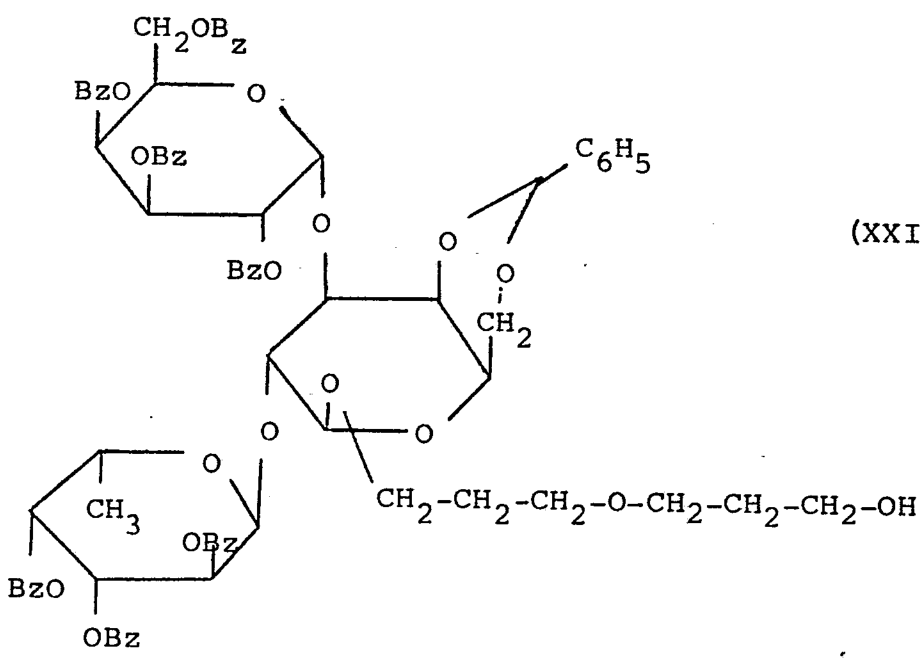

(XXIII)

à une réaction de tosylation, puis on fait réagir, au cours d'une deuxième étape b), le dérivé γ-tosylé obtenu avec un azide.

Le trisaccharide XXIII est avantageusement préparé selon le procédé décrit dans l'exemple 8 de la demande de brevet européen 79 400233.7 au nom de la demanderesse. Ce procédé comprend α) la réaction de l'allyl-3-O-benzoyl-2-O(2,3,4-tri-O-benzyl-α-L-fucopyranosyl)-4,6,O-benzylidène-β-D-galactopyranoside avec le 1-O-(N-méthyl)-acétimidyl-2,3,4,6-tétra-O-benzyl-β-D-galactopyranose ; β) la mise en oeuvre d'une réaction d'hydroboration sur le dérivé allyle, puis l'action d'une base ; γ la réaction du dérivé γ-hydroxypropyle ainsi obtenue avec un halogénure d'allyle qui est à son tour soumis à une réaction d'hydroboration.

Le trisaccharide XXIII est utilisé comme suit :

a) On ajoute 375 mg du trisaccharide de formule XXIII, dans 8 ml de chlorure de méthylène anhydre, 0,4 ml de triéthylamine, environ 3 mg de diméthylaminopyridine et 57 mg de chlorure de tosyle.

Après agitation du mélange 5 heures à température ambiante, on ajoute environ 20 ml d'eau glacée. On soumet encore le mélange réactionnel à agitation durant une heure et demie, puis on dilue avec 30 ml de chlorure de méthylène. On lave la solution réactionnelle successivement avec de l'acide chlorhydrique 2 M, de l'eau, du bicarbonate de sodium, puis de l'eau. On sèche sur du sulfate de sodium, puis on évapore la phase organique à sec. On récupère un produit amorphe renfermant le dérivé tosylé de l'alcool XXIII et on le soumet immédiatement au traitement suivant pour obtenir l'azide correspondant.

b) Le produit récupéré à l'étape précédente est dissous dans 8 ml de diméthylformamide. On ajoute 65 mg d'azide de sodium, puis on chauffe sous agitation à 80°C à

l'abri de l'humidité pendant 2 heures.

On refroidit le mélange réactionnel, puis on le verse dans 50 ml d'eau glacée. Après extraction avec 40 ml de chlorure de méthylène, on lave la phase extraite avec une solution de bicarbonate de sodium saturée, puis avec de l'eau et on la sèche sur du sulfate de sodium. On évapore le mélange réactionnel et on soumet le résidu à une chromatographie sur 15 g de silice.

On élue avec un mélange acétate d'éthyle/hexane 1/1 (vol/vol). On récupère 340 mg de produit sous forme de sirop, ce qui correspond à un rendement de 89 % pour les deux étapes.

Le sirop est recristallisé dans un mélange éther/hexane. On obtient 325 mg d'azide XXV (rendement 85 %) de P.F. 86-87°C, $[\alpha]_D^{20}$ = +3° (c 1, chloroforme), spectre RMN (chloroforme-d) = 1,17 (3H,d,J,6,5 Hz,Me-C) ; 5,40 (OH,5) ; 7,25 (40 H,m,Ph).

Analyse élémentaire : $C_{80}H_{89}N_3O_{16}$.

Calculé : C : 71,25 ; H : 6,65 ; N : 3,11

Trouvé : C : 71,05 ; H : 6,63 ; N : 3,14

EXEMPLE 2.

Préparation du γ[(γ-amino)propyloxy]-propyl-2-O-α-L-fucopyranosyl)-3-O-α-D-galactopyranosyl)-β-D-galactopyranoside, de formule XXVI :

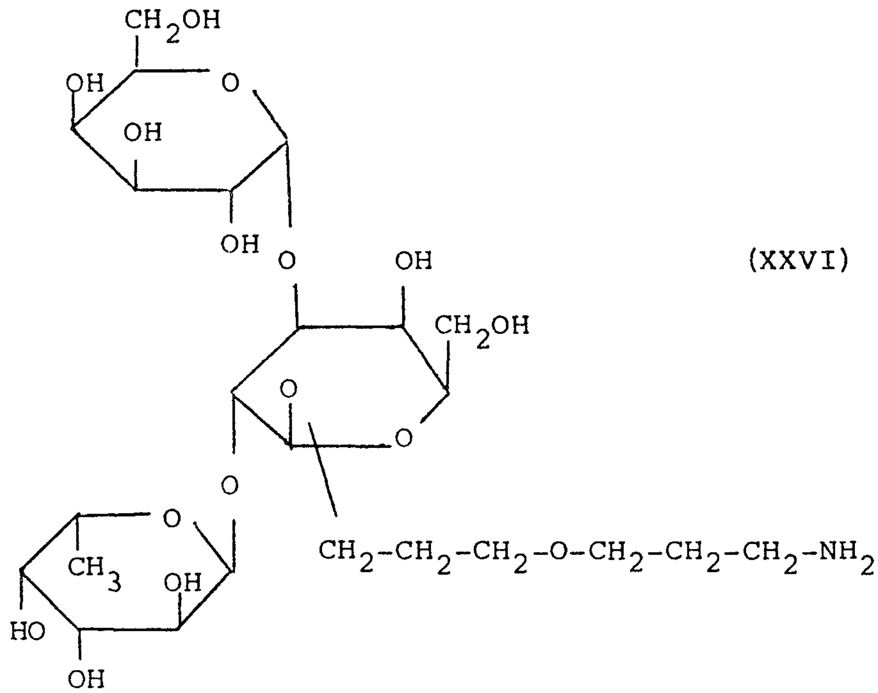

(XXVI)

On dissout 260 mg de l'azide de formule XXV dans 10 ml d'acide acétique en présence de 200 mg Pd/C 10 %.

On soumet le mélange à agitation sous atmosphère d'hydrogène durant 4 jours, puis on filtre. On évapore le filtrat à une température inférieure à 30°C. On ajoute de l'eau à plusieurs reprises, puis on évapore afin d'éliminer toute trace d'acide acétique.

On dissout le résidu d'évaporation dans 10 ml d'un mélange eau/éthanol (4/1), puis on soumet le mélange à agitation durant 10 mn sur une résine basique du type de celle commercialisée sous la dénomination Dowex 1 x 8. Après filtration, le mélange est lyophilisé. On récupère alors 107 mg d'une poudre hygroscopique (rendement 92 %). Ce produit brunit à 105°C et se décompose entre 176 et 180°C - $[\alpha_D^{20}]$= + 6° (c 1 eau/éthanol 1/1).

EXEMPLE 3.

Préparation d'un immunoabsorbant B par couplage du trisaccharide XXVI sur de la rafle de maïs.

On soumet tout d'abord la rafle de maïs à un traitement préliminaire en opérant comme suit :

On met en suspension la rafle de maïs dans 50 volumes de soude 4N. On soumet le mélange à agitation mécanique durant 8 heures et on laisse décanter. On siphonne alors la partie supérieure contenant les fines et on les jette.

La partie inférieure est filtrée sur verre fritté n° 2. On lave ensuite successivement avec de l'eau distillée jusqu'à neutralité, puis avec de l'acide chlorhydrique 1 N, puis avec de l'eau distillée jusqu'à neutralité, et jusqu'à l'obtention d'un produit qui ne relargue plus rien.

Le produit ayant subi ce prétraitement est ensuite activé à l'aide de carbonyldiimidazole : à cet effet, on met en suspension 2 g de rafle de maïs prétraitée et humide, puis on les filtre successivement dans les solutions suivantes :

- 2 fois 20 ml de dioxanne : eau (3:7)
- 2 fois 20 ml de dioxanne : eau (7:3)
- 2 fois 20 ml de dioxanne.

On les met ensuite en suspension dans une solution de 12 mg de carbonyldiimidazole dans 5 ml de dioxanne. On soumet le mélange à agitation pendant 15 minutes, puis on filtre et on lave la rafle avec 20 ml de dioxanne, à deux reprises.

On procède ensuite au couplage du trisaccharide XXVI. On dissout 0,6 mg de ce trisaccharide (900 nMoles) dans 10 ml d'eau. On ajuste le pH à 10 à l'aide de soude 1 N, puis on ajoute la rafle activée. On soumet le mélange à agitation à +4°C pendant 16 heures. On filtre et on rince avec 2 ml d'eau. On dispose d'un filtrat de 1,1 ml. Le dosage du trisaccharide XXVI dans le filtrat montre que celui-ci renferme 27 nMoles. $ml^{-1}$ de trisaccharide (soit 600 nMoles environ (900 - (27 x 11)) pour les 2 grammes de rafles utilisés). On a donc fixé 300 nMoles d'haptène par gramme. Afin de

bloquer les groupements actifs en excès, la rafle précédente est mise en suspension dans 5 ml de solution d'éthanolamine 1 M pH 8. Le mélange est soumis à agitation pendant 1 heure à la température ambiante, puis filtré sur verre fritté n° 2.

On lave, on filtre successivement avec l'eau un tampon phosphate 0,1 M pH 8,5 contenant 0,5 M de NaCl et un tampon acétate 0,1 M, pH 4,0 contenant 0,5 M de NaCl.

La rafle est mise en suspension dans du tampon tris 50 mM pH 7,0 contenant 0,02 % d'azide.

EXEMPLE 4.

Préparation d'un immunoabsorbant B par couplage du trisaccharide XXVI sur un support à base de polymère mixte d'agarose et d'acrylamide tel que ceux commercialisés par IBF sous les dénominations ULTROGEL et MAGNOGEL.

Les groupements -OH du support sont tout d'abord activés avec du bromure de cyanogène (BrCN) selon le mode opératoire décrit par Axen R., Porath J., Ernback S., dans Nature, 1967, 214, 1302.

On fixe ensuite le trisaccharide XXVI sur le support activé par BrCN en procédant comme suit :

On reprend le support activé par 1 volume de tampon bicarbonate de sodium 0,1 M pH 8,3 contenant 0,5 M de NaCl.

On ajoute au mélange 200 nMoles de trisaccharide XXV par gramme de support, puis on agite durant 2 heures à température ambiante.

On filtre et on lave le support sur lequel on a fixé le trisaccharide avec du tampon bicarbonate de sodium 0,1 M, pH 8,3 contenant 0,5 M de NaCl.

Afin de bloquer les groupements actifs en excès, on met le support en suspension dans 1 volume de solution d'éthanolamine 1 M, pH 8, pendant 2 heures à la température ambiante.

On complète avantageusement ce traitement comme suit pour saturer les charges :

On met en suspension le support précédent après lavage à l'eau dans une solution tampon phosphate 0,1 M pH 7,6 (1 vol) contenant soit 2 % de sérum albumine bovine de première qualité, soit 2 % de sérum albumine humaine, soit 2 % de sérum AB. Cette suspension est soumise à agitation pendant 2 heures à la température ambiante, puis filtré et lavé successivement par 10 volumes d'eau distillée, de solution de tampon phosphate 0,1 M pH 8,3 contenant 0,5 M de NaCl, puis de tampon d'acétate 0,1 M pH 4 contenant 0,5 M de NaCl.

On reprend enfin le mélange réactionnel par du tampon PBS pH 7,4. (Pour la conservation, on ajoute soit du merthiolate à 0,02 %, soit de l'azide de sodium à 0,02 %.

EXEMPLE 5.

Selon une variante, les supports de l'exemple 4 sont activés avec du carbonyldiimidazole. On opère à cet égard conformément à la méthode de Bethell G.S., Ayers J.S. et Hancock W.S., décrite dans J. Biol. Chem. 1979, 254, 2572.

Pour fixer le trisaccharide XXVI sur le support, on constitue, tout d'abord, une suspension du support activé dans 3 volumes d'eau distillée, puis on ajoute 200 nMoles de trisaccharide XXVI.

On ajuste le pH à 10. On agite 2 heures à la température ambiante, on filtre ensuite sur verre fritté n° 2 et on lave avec de l'eau distillée (10 volumes). Le blocage des groupements actifs en excès et le traitement complémentaire sont effectués conformément à l'exemple 4.

EXEMPLE 6.

Selon une autre variante, on procède à l'activation des groupements amide des supports définis dans

l'exemple 4, et ce à l'aide d'hydrazine ou de glutaraldéhyde. On met respectivement en oeuvre les méthodes décrites, d'une part, par Inman J.K. dans Meth. Enzymol. 1974, 34, 30 et Suttnar J. et al dans J. Chromatogr. 1977, 131, 453, et d'autre part par Weston P.D., Avrameas S., dans Biochem. Biophys. Res. Commun., 1971, 45, 1574.

On lave le support activé par 5 à 10 volumes d'eau distillée puis par 2 ou 3 volumes de tampon phosphate 0,5 M pH 7,6. Le gel est mis ensuite en suspension dans un égal volume de tampon phosphate 0,5 M pH 7,6. On ajoute alors 200 nMoles de trisaccharide XXV, puis on soumet le mélange à agitation 2 heures à température ambiante. On filtre sur verre fritté n° 2, puis on lave avec 10 volumes environ d'eau distillée.

Le blocage des groupements actifs en excès et le traitement complémentaire visant à saturer les charges sont réalisés comme décrit dans l'exemple 4.

EXEMPLE 7.

Préparation d'un immunoabsorbant B par couplage du trisaccharide XXVI sur un support Act-ULTROGEL-AcA 22 (il s'agit du support ULTROGEL AcA 22 activé par de la glutaraldéhyde).

3 x 1 ml de ULTROGEL AcA 22 sont lavés avec successivement :

- 2 x 20 ml d'eau distillée,
- 2 x 20 ml de tampon phosphate 0,5 M pH 8.

On constitue les trois suspensions renfermant 1 ml de support et 1 ml de tampon phosphate 0,5 M pH 8, en leur ajoutant respectivement 600 nM de trisaccharide XXVI, 300 nM et 150 nM.

On soumet à agitation ces trois fractions durant 16 heures à + 4°C, puis on les filtre sur verre fritté n° 2 et on dose les filtrats. Les filtrats n° 1, 2 et 3 contiennent respectivement 20 nM, 0 et 0. Donc, les

taux de trisaccharide fixé sur les fractions 1, 2 et 3 sont de 580, 300 et 150 nM. Afin de bloquer les groupements actifs en excès, on met les trois fractions en suspension dans 3 x 5 ml de solution 1 m d'éthanolamine 1 M, pH 8.

On soumet le mélange à agitation pendant 1 heure, on filtre et on lave successivement avec : 2 x 20 ml d'eau distillée ; 2 x 20 ml de tampon phosphate 0,1 M pH 8,3 contenant 0,5 M de NaCl et 2 x 20 ml de tampon acétate 0,1 M pH 4,0 contenant 0,5 M de NaCl.

Les supports sont ensuite mis en suspension dans du tampon tris 50 nM pH 7,0 contenant 0,02 % d'azide de sodium.

<u>EXEMPLE 8</u>.

Etude de la capacité d'absorption des immunoabsorbants B des exemples 6 et 3.

On utilise 1 ml de sérums-tests renfermant des anticorps anti-B (titre 1/32) pendant 2 heures à 22°C. On mesure l'agglutinabilité des anticorps anti-B avant et après absorption (mesure de la $HD_{50}$ hémagglutination) sur les immunoabsorbants de l'invention utilisés en quantités variables.

On rapporte les mesures en % de capacités d'absorption spécifique obtenues en fonction des quantités de trisaccharide que renferment les immunoabsorbants.

| Support | Déterminant antigénique en n-mole | % d'absorption |
|---|---|---|
| ULTROGEL | | |
| AcA 22 | 55 | 99,6 |
| | 30 | 97,5 |
| | 15 | 94,4 |
| MAGNOGEL | 55 | 95,4 |
| | 30 | 87,8 |
| | 15 | 87,4 |

Ces résultats mettent en évidence le pouvoir d'absorption spécifique élevé des immunoabsorbants renfermant un trisaccharide selon l'invention couplé sur un polymère d'agarose et d'acrylamide.

Les mesures effectuées dans les mêmes conditions avec la rafle de maïs montrent qu'avec des immunoabsorbants renfermant 15 nM, on obtient une absorption spécifique de l'ordre de 63 % et aucune absorption non spécifique.

EXEMPLE 9.

Application de l'immunoabsorbant selon l'exemple 3, 1°) a), à l'immunisation de lapins.

En suivant le protocole d'immunisation décrit par Martineau et al dans Immunochemistry 8, 705, 1971, on administre l'immunoabsorbant de l'exemple 3, 1°), a), avec de l'adjuvant complet de Freund à des lapins.

On prélève des échantillons de sang à divers instants et, par des essais quantitatifs de précipitine, selon les méthodes classiques, on contrôle le déroulement de l'immunisation.

On mesure des taux d'anticorps de 180 à 400 µg de sérum.

EXEMPLE 10.

Utilisation du trisaccharide de formule XXVI pour la neutralisation des hémolysines anti-B.

On utilise une solution à 10 mg/ml du produit de formule XXVI. En opérant selon les techniques de la pharmacopée européenne, on constate une inhibition totale d'une dose agglutinante d'un sérum-test anti-B avec une dilution 1/5 du trisaccharide de l'invention.

## REVENDICATIONS

1. Osides comprenant un motif galactopyranose dans lequel l'atome d'hydrogène du groupe -OH en position 1 est substitué par une chaîne A comprenant au moins un groupe éther intercalaire et un groupe fonctionnel azoté en bout de chaîne, dans lequel A est choisi dans le groupe constitué par un radical alcoxy-alcoylamine, alcoxy-alcoylamide ou alcoxy-alcoylazide dans lequel les groupes alcoxy et alcoyle comprennent chacun de 3 à 15 atomes de carbone, de préférence 4 à 10, avantageusement 6.

2. Osides selon la revendication 1, caractérisés en ce que les groupes alcoxy et alcoyle contiennent chacun 3 atomes de carbone, ou que le groupe alcoxy contient 3 atomes de carbone, tandis que le groupe alcoyle contient au moins 5 atomes de carbone.

3. Osides selon la revendication 1 ou 2, caractérisés en ce que A comprend en outre un ou plusieurs groupes insaturés, en particulier des radicaux éthyléniquement insaturés et/ou une substitution -OH et/ou des groupes intercalaires amino à la place ou en plus d'au moins l'un desdits groupes éther intercalaires.

4. Osides selon l'une quelconque des revendications 1 à 3, caractérisés en ce que ledit motif galactopyranose fait partie d'une structure trisaccharidique telle que celle du trisaccharide B, cette structure trisaccharidique répondant à la formule :

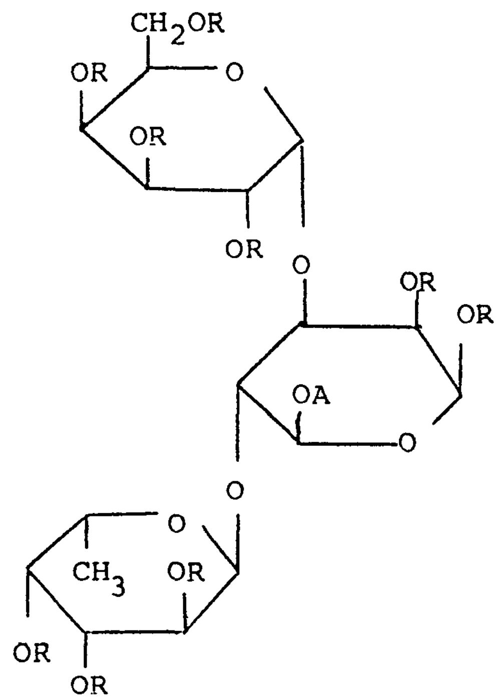

(XXVI)

dans laquelle A possède les significations données ci-dessus et les substituants R, identiques ou différents les uns des autres, représentent des groupements de protection de radicaux hydroxyle, le cas échéant avec un substituant voisin, et sont choisis parmi des groupes stables, non réactifs dans les conditions habituelles de synthèse osidique et facilement éliminables dans des conditions douces compatibles avec le maintien de la structure osidique, en particulier par des groupes constituant avec l'atome d'oxygène de l'hydroxyle des éthers benzyliques ou des acétals benzylidéniques.

5. Osides choisis dans le groupe constitué par le γ/(γ-azido)propyloxy7propyl-2-O-(2,3,4-tri-O-benzyl-α-L-fucopyranosyl)-3-O-(2,3,4,6-tétra-O-benzyl-α-D-galactopyranosyl)-4,6-O-benzylidène-β-D-galactopyranoside et le γ/(γ-amino)propyloxy7-propyl-2-O-α-L-fucopyranosyl)-3-O-α-D-galactopyranosyl)-β-D-galactopyranoside.

6. Procédé de préparation d'osides selon la revendication 1, caractérisé en ce qu'il comporte (1) une étape d'hydroboration selon laquelle on soumet l'oligo-

saccharide comportant un motif galactopyranose avec un groupement -OA, en position 1, dans lequel A représente un radical allyle à l'action d'un réactif tel que le 9-borabicyclo[3.3.1]nonane, puis à l'action d'une base, ce qui permet d'obtenir en position 1 une chaîne de substitution terminée par un groupement fonctionnel alcool, (2) la réaction de l'oside ainsi obtenu avec un halogénure d'allyle afin d'introduire un nouveau groupement allyle, que l'on soumet avantageusement à son tour à une nouvelle réaction d'hydroboration pour introduire un nouveau groupe allyle, et (3) après une réaction de tosylation, la réaction de l'oside obtenu avec un azide qui est le cas échéant réduit afin de conduire à l'amino-oside correspondant.

7. Application des osides selon l'une quelconque des revendications 1 à 5, pour la préparation d'un antigène artificiel B.

8. Application des osides selon l'une quelconque des revendications 1 à 5, sous forme libre ou immobilisée sur support, à la constitution de réactifs biologiques, en particulier pour la détection et la neutralisation d'hémolysines, ou à la préparation de sérums-tests présentant une spécificité donnée.

9. Application des osides selon l'une quelconque des revendications 1 à 5 à la préparation d'immunoabsorbants.

10. Immunoabsorbants comprenant au moins un oside selon l'une quelconque des revendications 1 à 5, et un support à base de polysaccharides, en particulier de cellulose, éventuellement en combinaison avec d'autres dérivés tels que la lignine.

11. Immunoabsorbants comprenant au moins un oside selon l'une quelconque des revendications 1 à 5, et de la rafle de maïs.

12. Immunoabsorbants comprenant au moins un oside selon l'une quelconque des revendications 1 à 5, et de

l'agarose ou de l'agarose modifié par réticulation de ses chaînes polysaccharidiques tel que celui commercialisé sous la marque "SEPHAROSE".

13. Immunoabsorbants comprenant un oside selon l'une quelconque des revendications 1 à 5, et des polymères mixtes, comprenant outre les polysaccharides définis dans la revendication 10 ou 12, d'autres dérivés polymères tels que des polyacrylamides, en particulier ceux commercialisés sous les marques "ULTROGEL" et "MAGNOGEL".

14. Immunoabsorbants selon l'une quelconque des revendications 10 à 13, comprenant un dérivé d'oside selon l'une quelconque des revendications 1 à 5 et un support solide, préalablement activé, par exemple à l'aide de bromure de cyanogène, de carbonyldiimidazole, d'hydrazine ou de glutaraldéhyde ou analogue.

15. Immunoabsorbants selon la revendication 11, caractérisés en ce que la rafle de maïs est activée avec du carbonyldiimidazole dans un solvant organique, avantageusement du dioxane, ce solvant étant avantageusement utilisé pour laver, au préalable, la rafle de maïs.

16. Immunoabsorbants selon l'une quelconque des revendications 10 à 15, tel qu'élaborés par couplage de 50 à 500 nM de dérivé d'oside par gramme de support, de préférence de 200 nM.

17. Immunoabsorbants selon l'une quelconque des revendications 10 à 16, caractérisés en ce que la fixation est réalisée en milieu basique en laissant les réactifs en contact pendant environ 12 à 24 heures.

18. Immunoabsorbants selon l'une quelconque des revendications 10 à 17, caractérisés en ce que le produit de couplage est traité pour bloquer les groupes réactifs du support qui n'ont pas réagi avec le dérivé d'oside, ce traitement comportant avantageusement l'utilisation d'un composé choisi dans le groupe constitué par de l'éthanolamine du tampon tris-HCl-pH8, de la glycine, de la D,L,lysine, de l'acide glutamique, de la glucosamine à 0,2M ph8 ou analogue, pour le bloquage des groupements réactifs du support, et qu'on ajoute avantageusement du NaCl O,5M.

19. Immunoabsorbants selon l'une quelconque des revendications 10 à 18, caractérisés en ce que le produit de couplage du dérivé d'oside avec le support est mis en contact avec une solution tampon contenant de la sérum albumine bovine ou humaine ou encore du sérum A,B et/ou est lavé successivement avec plusieurs tampons, tels qu'un tampon phosphate O,1 M de pH avantageusement de l'ordre de 8, de préférence entre 8 et 8,5 contenant O,5 M de NaCl puis un tampon acétate O,1M de pH inférieur à 5, avantageusement de l'ordre de 4, contenant O,5M de NaCl.

0038251

Office européen des brevets

# RAPPORT DE RECHERCHE EUROPEENNE

Numéro de la demande

EP 81 40 0547

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| | EP - A - 0 004 823 (ANVAR)<br>* Pages 28-33; pages 36-43 *<br>------- | 1-19 | C 07 H 3/06<br>3/08<br>15/18<br>G 07 N 33/80 |

DOMAINES TECHNIQUES RECHERCHES (Int. Cl.³)

C 07 H 3/06
3/08
15/18

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans *la demande*
L: document cité pour d'autres raisons

&: membre de la même famille, document correspondant

*Le présent rapport de recherche a été établi pour toutes les revendications*

| Lieu de la recherche | Date d'achèvement de la recherche | Examinateur |
|---|---|---|
| La Haye | 18-06-1981 | VERHULST |

OEB Form 1503.1 06.78